# FASCICULE DE BREVET EUROPEEN

(11) **EP 2 135 070 B1**
(45) Date de publication et mention de la délivrance du brevet: **19.06.2013**
(21) Numéro de dépôt: 08762107.4
(22) Date de dépôt: 18.02.2008
(51) Int. Cl.: G01N 33/08

(54) **MÉTHODE ET SYSTÈME DE RETASSAGE D'UN PLATEAU D'OEUFS**
VERFAHREN UND VORRICHTUNG ZUM AUFFÜLLEN EINER EIERTRAGPLATTE
METHOD AND SYSTEM FOR STABILISING AN EGG TRAY

(30) Priorité: 16.02.2007 FR 0753312
(43) Date de publication de la demande: 23.12.2009
(73) Titulaire: Egg-Chick Automated Technologies, 29400 Landivisiau (FR)
(72) Inventeur: MOGENET, Laurent, 33550 Libourne (FR); YVIN, Jean-Claude, 29250 Plougoulm (FR); DUPONT, Christophe, 29260 Le Folgoet (FR)
(74) Mandataire: Hays, Bertrand
(86) Numéro de dépôt international: PCT/FR2008/050259
(87) Numéro de publication internationale: WO 2008/110729

(56) Documents cités:
- WO-A-2006/078499
- FR-A- 2 858 919
- FR-A- 2 873 894
- US-A- 4 805 778
- US-A1- 2004 065 263
- US-A1- 2006 082 759

## Description

La présente invention concerne une méthode et un système automatisé de retassage de plateaux d'oeufs.

Dans l'industrie de la volaille, il est admis que 5 à 30% des oeufs ne sont pas fertiles ou viables ce qui va se traduire par l'absence de l'éclosion d'un poussin. Pour palier ce problème, des techniques permettant de discriminer, puis d'éliminer les oeufs fécondés (possédant un embryon) ou non fécondés ont été de développées. La solution technique actuellement la plus répandue pour réaliser la discrimination des oeufs fécondés est basée sur l'utilisation d'optique ou de diverses sources lumineuses et notamment le laser tel que décrit dans FR 0512998.

Cette étape de discrimination des oeufs est communément appelée « mirage » des oeufs. L'étape d'élimination des oeufs identifiés comme non fécondés à l'issue du « mirage » des oeufs est communément appelée « enlèvement » des oeufs.

La combinaison des étapes de « mirage » et d' « enlèvement » des oeufs permet d'obtenir une population homogène d'oeufs fécondés susceptibles d'éclore et de donner naissance à un poussin.

Les connaissances en embryologie ont rendu possible l'addition de divers composés bénéfiques aux poussins dans l'oeuf, directement chez l'embryon ou dans l'environnement proche de l'embryon. De tels composés bénéfiques peuvent se traduire notamment par la croissance accrue du poussin, par la prévention d'une maladie, par l'augmentation du pourcentage des oeufs éclos ou encore par l'amélioration des caractéristiques physiques de la volaille. L'addition de composés dans l'oeuf fécondé est communément appelée le traitement *in ovo.*

Dans le cas particulier de la prévention des maladies et plus particulièrement dans le cas de la vaccination, les techniques de traitement *in ovo* basées sur l'injection par aiguille du vaccin à travers la coquille de l'oeuf sont largement utilisées. Ainsi, un système d'injection permettant le traitement *in* ovo tout en réduisant l'effort sur les embryons provoqués par l'injection du vaccin est décrit dans le brevet FR1505870.

Pour des raisons économiques et afin d'éviter tout risque de contamination lié au traitement d'oeufs non fécondés (risque d'explosion et contamination via l'albumine contenue dans ces oeufs), il est préférable de réaliser le traitement *in ovo* sur des oeufs fécondés sélectionnés à l'issue d'une étape de mirage et d'enlèvement.

La viabilité économique du traitement *in ovo* réside principalement dans l'automatisation de l'ensemble des étapes de mirage, d'enlèvement et du traitement lui-même. Des solutions techniques d'intégration et d'automatisation de ces étapes ont été décrites dans le brevet FR2873894.

L'ensemble des solutions automatisées du mirage et de l'enlèvement des oeufs actuellement disponibles aboutit à l'obtention d'oeufs fécondés répartis de façon aléatoire et dispersés sur l'ensemble du plateau de présentation.

Pour s'adapter à cette caractéristique, des injecteurs particulièrement complexes et coûteux à mettre en oeuvre pour réaliser le traitement *in* ovo ont été développés (par exemple dans WO06/078499).

Le remplacement manuel des oeufs sur les plateaux de présentation pourrait être envisagé. En d'autres termes, un ou plusieurs opérateurs placeraient des oeufs dans les trous des plateaux desquels ont été retirés les oeufs non fécondés. Cette étape de remplacement est communément appelée « retassage ».

Cependant, cette alternative manuelle de « retassage » n'est pas compatible avec les cadences liées à l'automatisation du « mirage », de l' «enlèvement » et du traitement in *ovo* tel que réalisé actuellement.

Le but de l'invention est de résoudre ces problèmes.

La présente invention concerne une méthode et un système qui permet de remplacer automatiquement les oeufs éliminés suite aux étapes de « mirage » et d' « enlèvement » afin d'obtenir des plateaux d'oeufs fécondés répartis de façon homogène.

L'invention a ainsi pour objet une méthode automatisée de positionnement d'oeufs sur un plateau, comprenant, de manière automatisée, la détection de présence/absence d'oeufs dans les alvéoles d'un plateau, la préhension d'oeuf(s) dans une réserve et le placement dans des alvéoles vides du plateau.

Selon d'autres caractéristiques de l'invention :
- à l'aide une tête de préhension d'oeufs, l'on prélève les oeufs par groupe dans un plateau de réserve ou de retassage comportant un nombre d'alvéoles à oeufs qui est un multiple d'un groupe.
- l'on place l'ensemble des oeufs d'un groupe avant de retourner faire le prélèvement d'un autre groupe d'oeufs dans le plateau de réserve.
- l'on met en place les oeufs un par un, ou par groupe de plusieurs, en faisant se déplacer la tête de préhension d'oeufs en X et Y au-dessus du plateau à compléter ou à retasser.

D'autres caractéristiques de la méthode ressortiront de la description qui suit d'un système de positionnement d'oeufs.

L'invention a ainsi aussi pour objet un système de positionnement d'oeufs sur un plateau à retasser, le plateau comportant des alvéoles régulièrement réparties dans lesquelles des oeufs sont positionnés, une ou plusieurs alvéoles étant susceptibles de ne pas contenir un oeuf, cette dernière information étant enregistrée dans une unité de traitement d'informations, système dans lequel des moyens en forme de robot comprenant une tête de préhension d'oeufs, sont pilotés par l'unité de traitement d'informations pour prélever un ou des oeufs dans un plateau de réserve et les placer dans des alvéoles vides du plateau à retasser.

Selon d'autres caractéristiques de l'invention :
- le système comporte un convoyeur de déplacement du plateau sous des moyens de détection de présence/absence d'oeufs dans les alvéoles du plateau, jusqu'au poste de retassage.
- les moyens de détection se présentent sous la forme d'un portique équipé de capteurs placés au-dessus d'un convoyeur et sous lequel se déplace le plateau.
- la tête de préhension d'oeufs des moyens en forme de robot comporte des moyens de préhension d'un groupe de plusieurs oeufs.
- des moyens en forme de robot sont agencés de manière à pouvoir placer les oeufs dans les alvéoles du plateau à retasser un par un ou par groupe de plusieurs.
- les moyens en forme de robot sont agencés de manière à pouvoir prélever les oeufs par groupe à partir d'un plateau de réserve, chaque groupe correspondant à un sous-multiple du plateau de réserve ; la gestion du prélèvement permet de prélever de manière successive et par groupe, l'ensemble des oeufs placés sur le plateau de réserve.
- les moyens en forme de robot comprennent des moyens de déplacement de la tête de préhension en X et Y dans le poste de retassage.
- le système comporte un deuxième convoyeur de déplacement du plateau de réserve sous des deuxièmes moyens de détection de présence/absence d'oeufs dans les alvéoles du plateau, jusqu'au poste de retassage.
- ces deuxièmes moyens de détection se présentent sous la forme d'un portique équipé de capteurs placés au-dessus d'un convoyeur et sous lequel se déplace le plateau de réserve.
- le poste de retassage est dimensionné de manière à permettre aux moyens en forme de robot d'atteindre toutes les alvéoles du plateau à retasser et d'y placer un oeuf, quelle que soit la position de l'oeuf au niveau des moyens de préhension.
- les moyens de préhension sont actionnables par un ou plusieurs, en étant commandés par l'unité de traitement d'informations ; celle-ci est apte à gérer le dépôt de plusieurs oeuf simultanément en fonction des alvéoles vides placées dans le champ d'action des moyens de préhension en fonction du positionnement des moyens formant robot, en tenant compte des oeufs disponibles portés par ces moyens.

L'invention sera mieux comprise à la lecture de la description qui va suivre, donnée uniquement à titre d'exemple et faite en se référant aux dessins annexés, sur lesquels :

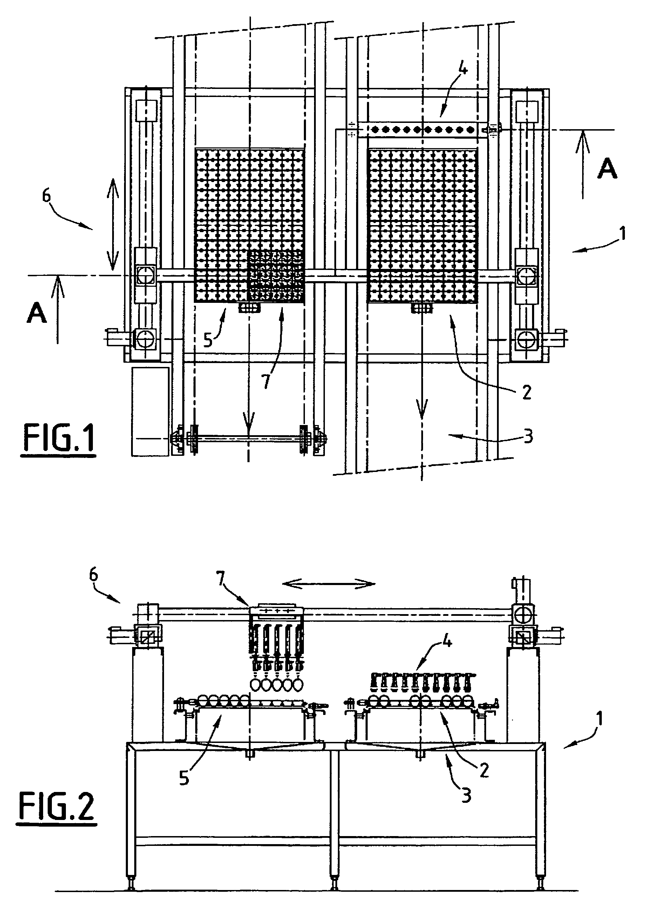

- la figure 1 représente une vue de dessus d'un système de retassage selon l'invention ; et
- la figure 2 représente une vue en coupe, prise suivant la ligne A-A de la figure 1.

On a en effet illustré sur ces figures, un système de retassage qui est désigné par la référence générale 1 de plateaux d'oeufs dont l'un est désigné par la référence générale 2, amenés à se déplacer sur un convoyeur ou un transporteur quelconque désigné par la référence générale 3 et présentant n'importe quelle structure appropriée.

En fait, un tel système peut être intégré dans une machine de traitement d'oeufs de type classique comme cela a été indiqué précédemment.

Lors de son entrée dans le poste de retassage, le plateau d'oeufs passe sous un portique désigné par la référence générale 4 sur ces figures, muni de moyens de détection de la présence ou de l'absence d'un oeuf dans chaque alvéole du plateau.

Différents moyens de réalisation de ces moyens de détection peuvent être envisagés, que ceux-ci soient optiques ou autres.

La détection est alors asservie de façon classique à la vitesse de déplacement du plateau pour obtenir une analyse complète et précise du plateau dans son entier et de chaque alvéole de celui-ci, afin de déterminer la présence ou l'absence d'un oeuf dans chaque alvéole du plateau.

En variante, ce dispositif de détection peut être omis, et l'information concernant la présence ou l'absence d'oeufs peut provenir d'une opération de mirage et d'enlèvement préalable. Il est cependant avantageux de prévoir ce dispositif de détection, car il permet de s'affranchir des risques de non enlèvement d'oeufs par suite d'un raté lors de cette étape préalable.

Une fois le plateau analysé en entier, ce plateau à retasser 2 arrive dans un poste de retassage dans lequel des moyens automatisés en forme de robot sont adaptés pour prélever des oeufs de remplacement dans un plateau de retassage ou de réserve désigné par la référence générale 5 sur ces figures, et pour venir regarnir, c'est-à-dire remplir, les trous vides du plateau à retasser.

A cet effet, le système comporte des moyens en forme de robot désignés par la référence générale 6 sur ces figures, comportant des moyens de déplacement en X et en Y d'une tête de préhension d'oeufs, désignée par la référence générale 7 sur ces figures, cette tête de préhension comportant des moyens de préhension de groupes d'oeufs dans le plateau de retassage 5 pour les disposer dans les alvéoles vides du plateau à retasser 2. Le plateau de retassage 5 comporte un nombre d'alvéoles qui est un multiple du nombre d'oeufs du groupe prélevé.

Ces moyens en forme de robot sont alors pilotés par un calculateur ou unité de traitement d'informations, également relié aux moyens de détection du portique 4 pour repérer le ou les emplacements vides du plateau à retasser. Les moyens sont également pilotés pour aller chercher successivement les groupes d'oeufs dans le plateau de retassage 5.

Dans l'exemple de réalisation décrit sur ces figures, la tête de préhension comporte des moyens de préhension d'un ensemble de vingt cinq oeufs répartis en cinq rangées de cinq colonnes et le plateau de retassage correspond à six groupes de vingt cinq oeufs.

Il va de soi bien entendu que différents modes de réalisation peuvent être envisagés.

C'est ainsi par exemple que les moyens formant robot 6 peuvent être actionnés par des moteurs électriques ou autres.

La tête de préhension 7 et les moyens de préhension des oeufs peuvent également présenter n'importe quelle structure appropriée munie d'un vérin de montée/descente de moyens à ventouse associés à des moyens d'aspiration, dont la structure est bien connue dans l'état de la technique pour manoeuvrer des oeufs de ce type.

Une fois le plateau à retasser rempli, celui-ci peut alors être amené vers un autre poste de la machine de traitement, à savoir par exemple un poste d'injection de vaccin dans ces oeufs.

Différents modes d'obtention des plateaux de retassage peuvent être envisagés.

Ainsi, par exemple, ceux-ci peuvent être remplis à la main ou encore être obtenus lors d'un passage d'un premier plateau sous les moyens de détection du portique 4.

On notera également que le plateau à retasser peut s'arrêter ou non en position dans le poste de retassage, par exemple en fonction du nombre de trous à compléter dans celui-ci.

On conçoit alors qu'un tel système présente un certain nombre d'avantages notamment au niveau des cadences de traitement des oeufs.

## Revendications

1. Système de positionnement d'oeufs sur un plateau à retasser (2), le plateau comportant des alvéoles régulièrement reparties dans lesquelles des oeufs sont positionnés, une ou plusieurs alvéoles étant susceptibles de ne pas contenir un oeuf, cette dernière information étant enregistrée dans une unité de traitement d'informations, lequel système comporte
- des moyens (6) en forme de robot comprenant une tête (7) de préhension d'oeufs et pilotés par l'unité de traitement d'informations pour prélever un ou des oeufs dans un plateau de réserve (5) et les placer dans des alvéoles vides du plateau à retasser (2),
- un convoyeur (3) pour déplacer le plateau à retasser jusqu'au poste de retassage,
**caractérisé en ce qu'**il comporte en outre des moyens (4) de détection de présence/absence d'oeufs dans les alvéoles du plateau (2) sous lesquels se déplace le plateau (2), lesdits moyens de détection étant reliés à ladite unité de traitement d'informations.

2. Système selon la revendication 1, **caractérisé en ce que** les moyens de détection (4) se présentent sous la forme d'un portique équipé de capteurs placés au-dessus d'un convoyeur et sous lequel se déplace le plateau (2).

3. Système selon la revendication 1 ou 2, **caractérisé en ce que** la tête (7) de préhension d'oeufs des moyens en forme de robot comporte des moyens de préhension d'un groupe de plusieurs oeufs.

4. Système selon la revendication 3, **caractérisé en ce que** des moyens en forme de robot sont agencés de manière a pouvoir placer les oeufs dans les alvéoles du plateau à retasser un par un ou par groupe de plusieurs.

5. Système selon la revendication 3 ou 4, **caractérisé en ce que** les moyens en forme de robot sont agencés de manière a pouvoir prélever les oeufs par groupe à partir d'un plateau de réserve (5), chaque groupe correspondant à un sous-multiple du plateau de réserve (5).

6. Système selon l'une quelconque des revendications 1 à 5, **caractérisé en ce que** les moyens en forme de robot (6) comprennent des moyens de déplacement de la tête de préhension (7) en X et Y dans le poste de retassage.

7. Système selon l'une quelconque des revendications 1 à 5, **caractérisé en ce qu'**il comporte un deuxième convoyeur de déplacement du plateau de réserve (5) sous des deuxièmes moyens de détection de présence/absence d'oeufs dans les alvéoles du plateau (5), jusqu'au poste de retassage.

## Patentansprüche

1. System zur Positionierung von Eiern auf einer Eiertragplatte (2), wobei die Platte regelmäßig verteilte Waben umfasst, in denen die Eier positioniert werden, wobei eine oder mehrere Wagen kein Ei enthalten können, wobei diese letztgenannte Information in einer Informationsverarbeitungseinheit gespeichert wird, wobei das System Folgendes umfasst:
- Mittel (6) in Form eines Roboters, umfassend einen Kopf (7) zum Ergreifen von Eiern und gesteuert von der Informationsverarbeitungseinheit, um ein ober mehrere Eier aus einer Reserveplatte (5) zu entnehmen und sie in leeren Waben der Eiertragplatte (2) anzuordnen,
- einen Förderer (3), um die Eiertragplatte zur Auffüllstation zu verschrieben,
**dadurch gekennzeichnet, dass** es ferner Mittel (4) zur Erfassung des Vorhandenseins/Fehlens von Eiern in den Waben der Platte (2) umfasst, unter denen sich die Platte (2) verschiebt, wobei die Erfassungsmittel mit der Informationsverarbeitungseinheit verbunden sind.

2. System nach Anspruch 1, **dadurch gekennzeichnet, dass** die Erfassungsmittel (4) in Form eines Portalrahmens vorhanden sind, der mit Sensoren, die über einem Förderer angeordnet sind, ausgestattet ist, und unter dem sich die Platte (2) verschiebt.

3. System nach Anspruch 1 oder 2, **dadurch gekennzeichnet, dass** der Kopf (7) zum Ergreifen von Eiern der Mittel in Roboterform Mittel zum Ergreifen einer Gruppe von mehreren Eiern umfasst.

4. System nach Anspruch 3, **dadurch gekennzeichnet, dass** die Mittel in Roboterform derart angeordnet sind, dass sie die Eier in den Waben der Eiertragplatte einzeln oder in Gruppen zu mehreren anordnen können.

5. System nach Anspruch 3 oder 4, **dadurch gekennzeichnet, dass** die Mittel in Roboterform derart angeordnet sind, dass sie die Eier in einer Gruppe aus einer Reserveplatte (5) entnehmen können, wobei jede Gruppe einem Untervielfachen der Reserveplatte (5) entspricht.

6. System nach einem der Ansprüche 1 bis 5, **dadurch gekennzeichnet, dass** die Mittel in Roboterform (6) Mittel zum Verschieben des Greifkopfes (7) in X- und Y-Richtung in der Auffüllstation umfassen.

7. System nach einem der Ansprüche 1 bis 5, **dadurch gekennzeichnet, dass** es einen zweiten Förderer zum Verschrieben der Reserveplatte (5) unter zweiten Mitteln zur Erfassung des Vorhandenseins/Fehlens von Eiern in den Waben der Platte (5) bis zur Auffüllstation umfasst.

## Claims

1. System for positioning eggs on a stabilising tray (2), the tray comprising evenly distributed cells in which eggs are positionned, one or more cells being likely not to contain eggs, this information being stored in an information processing unit, which system comprising:
- means in the form of a robot (6) comprising an egg pick-up head (7), driven by the information processing unit in order to pick up one or more eggs from a supply tray (5) and place them in empty cells of the stabilizing tray (2),
- a conveyor (3) for moving the tray to the stabilising station,
**characterized in that** it further comprises means (4) for detectecting the presence/absence of eggs in the cells of tray (2) whereunder the tray moves, said detection means being connected to said information processing unit .

2. System according to claim 1, **characterized in that** the detection means (4) are shaped in the form of a gantry fitted with sensors arranged over a conveyor and whereunder the tray (2) moves.

3. System according to claim 1 or 2, **characterized in that** the egg pick-up head (7) of the means in the form of a robot comprises means for picking-up a group of plural eggs.

4. System according to claim 3, **characterized in that** the means in the form of a robot are arranged so as to make it possible to place the eggs one by one or by groups in the cells of the stabilising tray.

5. System according to claims 3 or 4, **characterized in that** the means in the form of a robot are arranged so as to make it possible to pick up the eggs by group from a supply tray (5), each group corresponding to a sub-multiple of the supply tray (5).

6. System according to any one of claims 1 to 5, **characterized in that** the means in the form of a robot (6) comprise means for moving the egg pick-up head (7) in an X and Y directions in the stabilising station.

7. System according to any one of claims 1 to 5, **characterized in that** it comprises a second conveyor for moving the supply tray (5), under second detection means for detecting the presence/absence of eggs in the cells of tray (5), to the stabilising station.
